# EUROPEAN PATENT SPECIFICATION

(11) **EP 4 157 102 B1**
(45) Date of publication and mention of the grant of the patent: **15.04.2026**
(21) Application number: 21734625.3
(22) Date of filing: 27.05.2021
(51) Int. Cl.: A61B 17/04, A61B 17/00, A61B 17/06

(54) **TISSUE REPAIR SYSTEM**
GEWEBEREPARATURSYSTEM
SYSTÈME DE RÉPARATION DE TISSU

(30) Priority: 27.05.2020 US 202063030508 P
(43) Date of publication of application: 05.04.2023
(73) Proprietor: Smith & Nephew, Inc., Memphis, TN 38116 (US); Smith & Nephew Orthopaedics AG, 6300 Zug (CH); Smith & Nephew Asia Pacific Pte. Limited, Singapore 138565 (SG)
(72) Inventor: ROGERS, Jon-Paul, Mansfield, Massachusetts 02048 (US); QI, Zenan, Mansfield, Massachusetts 02048 (US); HOUSMAN, Mark Edwin, Mansfield, Massachusetts 02048 (US); PATEL, Nehal Navinbhai, Mansfield, Massachusetts 02048 (US); HALL, Benjamin Michael, Mansfield, Massachusetts 02048 (US); SANTANGELO, Stephen Anthony, Andover, Massachusetts 01810 (US); THYDEN, Michael, Andover, Massachusetts 01810 (US)
(74) Representative: Appleyard Lees IP LLP
(86) International application number: PCT/US2021/034590
(87) International publication number: WO 2021/243062

(56) References cited:
- US-A1- 2003 204 195
- US-A1- 2010 069 923
- US-B2- 7 645 293
- US-B2- 9 962 149

## Description

### FIELD

The present disclosure relates to methods and devices for tissue repair. More specifically this disclosure relates to an anchor construct that may be a soft anchor, used for tissue repair in patient extremities.

### BACKGROUND

Tissue repair may require coupling bone to a repair tissue using anchors and flexible members. Repair tissue may include soft tissue, another bone or another segment of the same bone. Bone anchors with flexible members attached thereto may be coupled to the bone. In order to complete the tissue repair, the bone may then be coupled to the repair tissue via the flexible member. This may require coupling a threading member such as a needle to the flexible member or coupling a second anchor to the flexible member, which can be time consuming. Furthermore, for procedures in extremities, such as on the hand or foot, coupling means such as threading members or anchors preferably have a smaller profile to minimize trauma to these finer more delicate tissues; tissues such as an anterior talofibular ligament (ATFL) ligament. Providing threading members or second anchors pre-attached may save time, especially when considering coupling to a smaller profile coupling means. However, managing these multiple components may become overly complicated. Therefore, there is a need for an improved tissue repair system with pre-assembled lower profile coupling means for coupling to smaller or more delicate tissues of the hand or foot. There is also a need for an improved tissue repair system that manages the stages of tissue repair.

US 7 645 293 B2 is directed to an apparatus for placement of a suture anchor having a suture connected thereto. The apparatus includes a handle dimensioned for engagement by the user and an elongated member connected to the handle and extending therefrom. The elongated member has an anchor mount for mounting a suture anchor. The handle includes a frame having a suture retainer adapted for retaining a suture and at least one cover releasably mounted to the frame to at least partially enclose the suture retainer. A release button may be mounted to the frame and is movable to release the at least one cover from the frame to expose the suture portion. US 2003/204195 A1 and US 2010/069923 A1 relate to further suture anchor deployment devices with a storage capacity for a surgical suture within a housing of the suture anchor deployment devices.

### SUMMARY

The present invention is defined by the features of the independent claim. Embodiments of the invention are defined in the dependent claims.

Disclosed herein is a tissue repair system that may improve tissue repair that may include coupling two tissues. These tissues may include a first tissue and a second tissue, or two segments of the same tissue. For example, the first tissue may be bone and the second tissue may be a soft tissue. The system may include a repair construct including a tissue anchor, a coupling means and a flexible repair member coupling the two. The system may include an instrument that houses the repair construct and sets up a staged deployment or release of the different portions of the repair construct according the tissue repair technique, thereby simplifying the repair steps.

An example tissue repair system disclosed herein includes an instrument including a handle at a proximal end, a shaft extending distally from the handle and a housing with a cover. The instrument may manage a tissue repair construct for coupling a first tissue to a second tissue. Tissue repair construct includes a tissue anchor. Tissue anchor is disposed at a distal end of the shaft and is operatively coupled to an actuation member of the handle via a deployment member. Deployment member extends along the shaft. Tissue repair construct also includes a repair member that couples the tissue anchor to the second tissue. The repair member has a first end operatively coupled to the tissue anchor and second end operatively coupled to at least one coupling means. The second end and coupling means is housed within the instrument housing. Actuation of the actuation member is configured to deploy the tissue anchor via the deployment member and thereby couple the tissue anchor with the first tissue. Actuation of the actuation member also releases the housing cover for access to the coupling means.

In some example embodiments, the shaft has a first position and second position, wherein in the first position the shaft is operatively coupled to the housing cover and locks the housing cover and wherein actuation of the actuation member moves the shaft to the second position that unlocks the housing cover. In some example embodiments, the shaft distal end may house the tissue anchor therein, and actuation of the actuation member axially moves the shaft to expose the tissue anchor. In some example embodiments, the housing cover defines an external circumferential surface the instrument. In some example embodiments, the housing cover includes a slot for engaging the repair member, such that removing the housing cover removes a portion of the repair member. In some example embodiments, the coupling means is selected from a group comprising a needle, a threading member, a cortical button and another tissue anchor. In some example embodiments, the tissue anchor is a soft anchor and the deployment member includes a flexible member repeatedly interwoven through the soft anchor. The flexible member may define a first cross section and the repair member may be another flexible member with a second different cross section, smaller than the first cross section. The flexible member may include a frangible portion that fractures at a predetermined value indicative of tissue anchor deployment, such that the flexible member may then be removed leaving the tissue anchor deployed within the first tissue. In some example embodiments, the repair member includes at least two flexible members, defining at least four flexible member limbs, each limb terminating with a coupling means that is a needle.

An example method of securing a first tissue to a second tissue is also disclosed including inserting a tissue anchor into the first tissue with an instrument, the instrument including a handle at a proximal end, a shaft extending distally from the handle and a housing having a cover, locked to the instrument. An actuation member of the instrument is then actuated to deploy the tissue anchor and couple the tissue anchor with the first tissue. Actuating also unlocks the housing cover. The housing cover is then removed from the instrument to expose a coupling means and a repair member disposed within the housing. The repair member is operatively coupled to the tissue anchor. The repair member is then coupled to the second tissue via the coupling means.

In some example methods, actuating the actuation member of the instrument axially moves the instrument shaft through an opening of the housing cover to unlock the housing cover. Some example methods also include removing the coupling means from a slot in the housing cover to release the coupling means from the housing cover before coupling the repair member to the second tissue. In some example methods, the repair member is removed from retaining slots within the housing before coupling the repair member to the second tissue. In some example methods, the coupling means includes at least one needle and coupling the repair member to the second tissue via the coupling means includes inserting the at least one needle through the second tissue. In some example methods, the tissue anchor is a soft anchor operatively coupled to the actuation member via a flexible member and actuating the actuation member applies tension on the flexible member and deploys the soft anchor. The flexible member may include a frangible portion and actuating the actuation member further comprises actuating the actuation member to fracture the flexible member after deploying the soft anchor. In some example methods, the repair member includes at least two flexible members, defining at least four flexible member limbs, each limb terminating with a coupling means that is a needle.

Another example tissue repair system is disclosed, for securing a first tissue to a second tissue. It includes an instrument with a handle at a proximal end, a shaft extending distally from the handle and a housing with a removable cover. A tissue anchor is housed within a distal end of the shaft, the tissue anchor operatively coupled to an actuation member of the handle via a deployment member. A flexible member is coupled to the tissue anchor at a first end of the tissue anchor. A second end of the flexible member is operatively coupled to at least one coupling means. The second end and coupling means are housed within the instrument housing. Actuation of the actuation member deploys the tissue anchor via the deployment member and thereby couples the tissue anchor with the first tissue. It also axially moves the shaft and unlock the housing cover for access to the coupling means. In some example embodiments, the coupling means is selected from a group comprising a needle, a threading member, a cortical button and another tissue anchor.

### BRIEF DESCRIPTION OF THE DRAWINGS

The disclosure will be more fully understood by reference to the detailed description, in conjunction with the following figures, wherein:
FIG. 1 illustrates a tissue repair system in accordance with this disclosure;
FIG. 2 illustrates a distal end of a tissue repair system in accordance with this disclosure;
FIGS. 3A-3C illustrate a method of tissue repair in accordance with this disclosure;
FIG. 4 illustrates components of an insertion instrument of a tissue repair system in accordance with this disclosure;
FIG. 5 illustrates a cross section of an insertion instrument in accordance with this disclosure;
FIGS. 6A and 6B illustrate a method of deploying and unlocking a cover of a tissue repair system, in accordance with this disclosure;
FIGS. 7A and 7B illustrate various views of hub covers, in accordance with this disclosure;
FIG. 8 illustrates another example hub cover, in accordance with this disclosure;
FIG. 9 illustrates a kit for tissue repair, in accordance with this disclosure;
FIGS. 10A-10I illustrate a method of tissue repair with the kit illustrated in FIG. 9, in accordance with this disclosure;
FIGS 11A and 11B illustrate example embodiments of hub cover attachment, in accordance with this disclosure;
FIG. 12 illustrates a frangible deployment member, in accordance with this disclosure;
FIGS. 13A-13C illustrates a method of tissue repair with a frangible deployment member, in accordance with this disclosure; and
FIG. 14A-14B illustrates an example tissue repair construct including a second tissue anchor, in accordance with this disclosure.

### DETAILED DESCRIPTION

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

As used in the specification and claims, for the purposes of describing and defining the invention, the terms "about" and "substantially" are used to represent the inherent degree of uncertainty that may be attributed to any quantitative comparison, value, measurement, or other representation. The terms "about" and "substantially" are also used herein to represent the degree by which a quantitative representation may vary from a stated reference without resulting in a change in the basic function of the subject matter at issue. "Comprise," "include," and/or plural forms of each are open ended and include the listed parts and can include additional parts that are not listed. "And/or" is openended and includes one or more of the listed parts and combinations of the listed parts. Use of the terms "upper," "lower," "upwards," and the like is intended only to help in the clear description of the present disclosure and are not intended to limit the structure, positioning and/or operation of the disclosure in any manner.

Disclosed herein is a tissue repair system that includes an instrument for placing and fixing a first tissue anchor with a first tissue, hereafter defined as deployment of the anchor. The tissue anchor may be coupled to at least one flexible member that may have coupling means such as needles, threading members or second tissue anchors attached thereto. First and second tissue anchors may be different from each other. Coupling means may be attached to the at least one flexible member at a location spaced away from the first tissue anchor and the position of the coupling means may be fixed or adjustable. The coupling means may be used to couple the at least one flexible member to a second tissue. Flexible members may include suture, suture tape, cable, a spring or wire for example. The insertion instrument includes a means of managing and staging release of the flexible member and coupling means. The insertion instrument may include a means of concealing or retaining the flexible member and coupling means, as they may not be needed until after deploying the first tissue anchor with the first tissue. The insertion instrument may include a means of housing and protecting the at least one flexible member and coupling means until after or during anchor deployment. The insertion instrument may include a means of staging the release of the flexible member and coupling means, to aid in managing components of the tissue repair according to the methods of use.

The first tissue anchor may be a soft anchor formed of a flexible material. The first tissue anchor may include braided suture or flexible lengths of tape in a form that changes shape via forces from deployment member. This change in shape may include changing from an elongate configuration for inserting into a first tissue such as bone, to a deployed expanded form whereby the soft anchor engages walls of the first tissue and anchors or fixes the soft anchor with the bone. The soft anchor in the deployed state resists begin pulled out to the tissue into which it has been inserted. Deployment may occur via tension on a deployment member that is operatively coupled to the soft anchor. Deployment member may be a flexible member and may be suture, wire, ribbon, cable, spring or suture tape. Deployment may be controlled via an insertion instrument that is configured to house or couple to the soft anchor, insert the anchor into (and maybe through) the first tissue and then control the deployment member to deploy the soft anchor. The soft anchor may be deployed by controlled tension to a target tension value. Soft anchor may be similar to the anchoring system disclosed in at least U.S. Publication No. 2013/0123810 (Brown et al.), and U.S. Publication 2019/0247039 (Gregoire et al), both commonly assigned are referenced herein.

In the example soft anchor, deployment member may include an expansion or deploying flexible member in the form of a suture that may be repeatedly interwoven through the soft anchor. The expansion suture extends along the instrument to couple to an actuation means portion of the instrument handle. Tensioning the expansion suture via the actuation means changes the soft anchor configuration to create an expanded deployed configuration. Tensioning the expansion suture may include rotating a wheel of the handle, as disclosed in at least U.S. Publication No. 2013/0123810 (Brown et al.). The expansion suture may then be disconnected from the actuation means and passed through a small cannulation of the instrument to release the inserter instrument from the expansion member and soft anchor. This expansion suture may then be coupled to coupling means such as a needle; threading member or second anchor to attached the soft anchor to a second tissue via the expansion suture and complete the tissue repair.

As explained earlier, threading a needle or attaching a suture to a second anchor is time consuming, especially with the evolution of ever-shrinking medical device sizes. This can add time to the procedure and is not ideal for small tissues. Expansion suture may also be relatively large in diameter or width, required to withstand the higher tension loads required for anchor deployment, these larger diameters not suited for repair of smaller more delicate tissues. Therefore, a separate repair member operatively coupled to the first anchor, such as a separate flexible member may be provided for coupling the first tissue to the second tissue. This repair member may include the coupling means pre-assembled, thereby overcoming the complexity of attaching the coupling means. This repair member may preferably be smaller in diameter or width than a deployment member, for coupling to smaller or more delicate tissues. The system may therefore include multiple members such as a deployment member and a repair member, each having separate functions and for use during different stages of the tissue repair.

For example, when used in mini-open procedures, typical for procedures in the extremities such as the ankles, feet or hands, having pre-attached needles allows the surgeon not avoid threading the repair suture through an open needle eyelet. Preferably, these repair members are small in size (#2-0, #0 or #1 suture). Having a repair member with needles pre-attached allows the surgeon to more accurately repair these small ligament/tendons and small tissue, and may allow for repeatedly passing the needle through the tissue. Pre-attaching these needles may be via crimping, to keep the attachment profile minimal. Crimping may provide a lower profile attachment than an eyelet for example, preferable for repair of smaller or more fragile tissues.

In some embodiments, the deployment member may be removable once the anchor is deployed and a tissue is coupled to the anchor. In some embodiments a third flexible member may also operatively couple to the soft anchor to lock or bind the system and prevent the anchor from loosening and may be termed a locking member. In some example embodiments, a single flexible member may perform at least two of these tasks. For example, a single member may both act as the locking member and repair member.

Disclosed herein is a tissue repair system that includes an insertion instrument and a flexible member with coupling means attached thereto, the coupling means housed within the inserter. Disclosed herein is a system that helps manage the coupling means. The coupling means is coupled to a repair member. The repair member may also function as a locking member. The insertion instrument may include a means to selective release or expose the coupling means from the inserter, as well as the repair member attached thereto. The system may have an inserter with a working length preferable for mini-open procedures typical for surgery on the extremities. For example, an inserter shaft length may be less than 12,7cm (5 inches), and may more preferable be approximately 7,62cm (3 inches). Contrary to surgeries for example on the hip, where inserters may have a shaft length of between 15,24 - 20,32cm (6-8 inches), this longer length shaft is challenging to control during an extremities procedure, as the inserter shaft is not stabilized by a long length of tissue as is the case of the tissue around the hip for example. Disclosed herein is a tissue repair system that may include two repair members and management thereof. The second repair member may also include coupling means and may provide the surgeon with the opportunity for additional tissue repair per anchor.

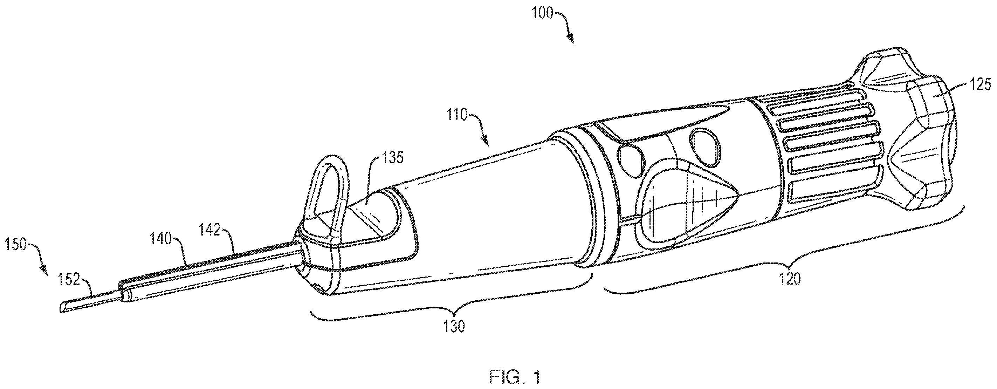

Referring now to FIG. 1, an example tissue repair system 100 according to the disclosure is illustrated. System 100 includes an insertion instrument 110 having a handle 120 with actuation means 125 for deploying the anchor (not shown in this figure). Anchor may be operatively coupled to a distal end 150 of the instrument 110. Hub 130 extends between handle 120 and shaft 140, the shaft extending from the hub 130 to the distal end 150. Hub 130 includes a cavity (shown in more detail later) for housing at least one coupling means such as needles, threading members or anchors, Cavity also houses a portion of at least repair member that is attached to the coupling means. Distal end 150 includes a slotted tube 152, the slot 153 configured to allow passage of the repair member(s) therethrough. Shaft 140 includes an elongate channel having an open lateral side to house the repair member therein. Therefore, the repair member(s) extends from the within the distal end 150, where it is operatively coupled to the anchor, and extends through slot 153, into and along elongate channel 142 and into the cavity. Hub 130 includes a removable cover 135 that may be selectively unlocked and removed to expose the coupling means and repair member attached thereto.

Tissue repair system 100 may deploy a soft anchor as disclosed herein, via tension on a deployment member, similar to the Q-fix system offered by Smith and Nephew. In this example system, deployment member may include a flexible member such as a suture that operatively couples to a soft anchor, housed within the slotted tube 152 at the distal end 150. In this example, deployment member is operatively coupled to a tension actuation means 125. Deployment member may be a length of suture that may be spooled around a shaft of the actuation means 125, and turning of actuation means 125 around a longitudinal axis of handle may wind the suture and apply tension sufficient to deploy the anchor.

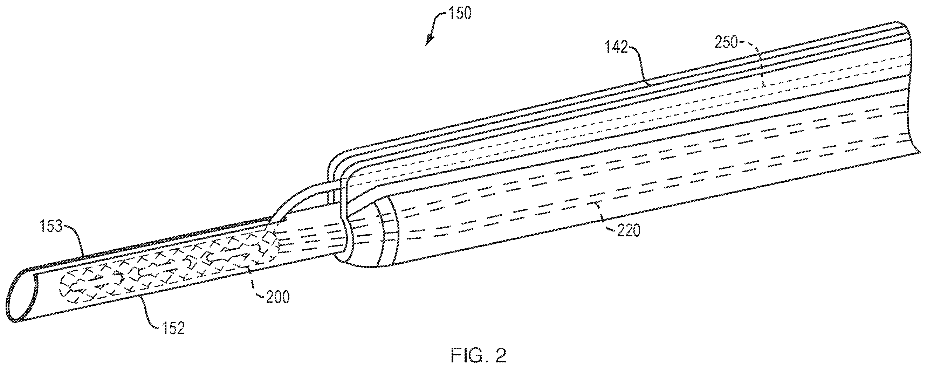

FIG. 2 illustrates instrument distal end 150 with anchor 200 disposed along slotted tube 152. Slot 153 extends from the distal most edge of slotted 152 and extends partially along a long axis of the tube 152. Slot 153 may have a proximal most edge adjacent a corresponding proximal edge of the anchor 200 when the anchor 200 is in the elongate or non-deployed configuration. Deployment member 220 may interweave through anchor 200 and extend along the shaft 140. Deployment member 220 may extend along an inner lumen of shaft 140. Anchor 200 is also operatively coupled to at least one repair member 250, different from deployment member 220 that may extend through slot 153 and into an externally disposed channel 142. Channel 142 is continuous with cavity formed within hub 130. For simplification purposes, repair member 250 is shown in FIG. 2 as coupled to a proximal end of anchor 200. Coupling of repair member 250 to anchor 200 may via attaching means such as glue, adhesive, knots, splices, heat treatment or staples. Alternatively or in addition to, repair member 250 may interweave through anchor 200 in a similar manner to deployment member 220. Repair member 250 may interweave partially or along the entire length of the anchor 200. Repair member 250 may interweave along the same path as deployment member 220, or take a different weave pattern therealong and through the anchor thickness, partially or along the entire length of the anchor 200. Repair member 250 may interweave along a path parallel to the deployment member 220 but along a plane that is rotated about the anchor longitudinal axis at a non-zero angle. Stated alternatively, repair member 250 may interweave along a path parallel to the deployment member 220 and circumferentially offset from the deployment member path through the anchor 200. Repair member 250 may interweave along a path parallel to the deployment member 220 that is also along a plane angled at about 90 degrees from the deployment member path through the anchor 200.

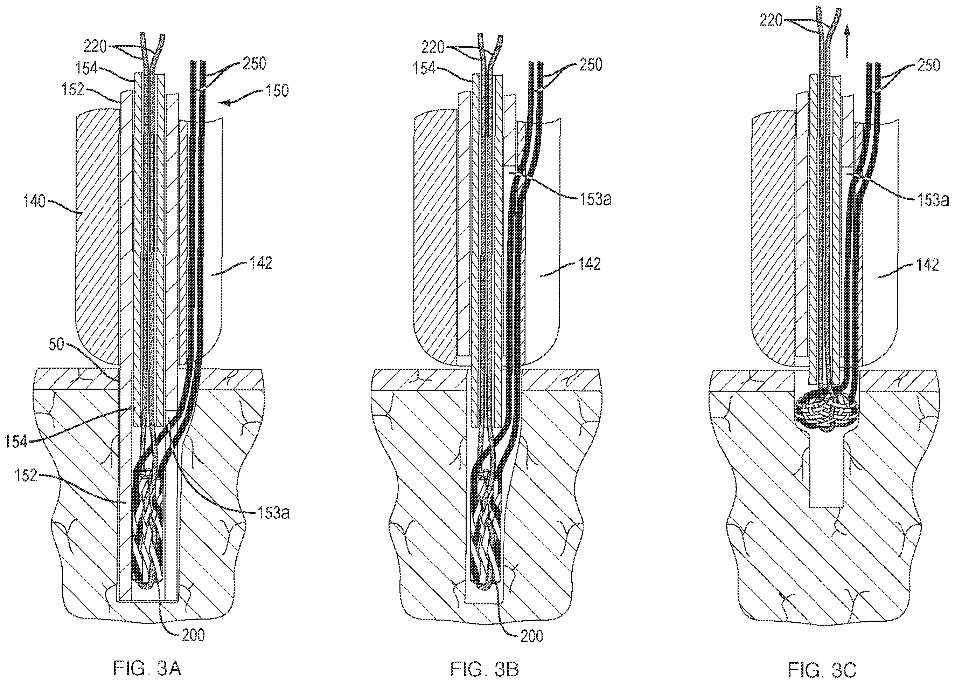

FIG. 3A, 3B and 3C illustrate details of the distal end 1 50 and stages of deployment. FIG. 3A illustrates the distal end 150 as initially inserted into a tissue hole 50, with anchor 200 housed within slotted tube 152. Of note, the right side of tube in this figure shows proximal end 153a of slot 153. The slotted tube 152 may be pushed into the tissue hole 50. An anchor pusher or backstop 154, coaxial with slotted tube 152 and also at least partially may enter tissue hole 50 and remains close to or touching the proximal end of the anchor 200.

Turning now to FIG. 3B, another cross section view is provided showing the subsequent step to FIG. 3A in an example use of the tissue repair system 100. The anchor 200 is shown placed into the hole 50 in a coaxial orientation. During deployment, slotted tube 152 is retracted in an axial direction while the anchor pusher 154 remains in place. Slotted tube 152 is operatively coupled at its proximal end to the actuation means 125. Therefore actuation of the actuation means 125 may perform at least two operations; it may retract slotted tube 152 to expose or release the anchor 200 into the hole and also apply tension to the deployment member to deploy the anchor 200. The two operations may be sequentially spaced, or at least partially overlap such that some but not all retraction of the tube 152 may occur before tension is initiated on the deployment member 220. The anchor pusher 154 may be fixedly connected to the proximal end to the handle 120. Tissue hole 50 may be pre-formed using a drill. Tissue may be bone.

FIG. 3C illustrates the anchor 200 in a fully deployed state. The anchor pusher 154 ideally extends into the tissue, and when the tissue is bone tissue, the anchor pusher has a distal edge that is adjacent the bottom of the cortical layer, typically 0,508 - 1,27mm (0.02"-0.05") below the botie/tissue surface. As the deployment member 220 is tensioned, the anchor 200 retracts upon itself with the end of the anchor pusher 154 providing counter traction and assumes a shorted, expanded state with an increased effective diameter. By "counter traction," we mean a backstop is provided resisting movement of the proximal end of the anchor proximally, thus causing the anchor 200 to bunch up and expand.

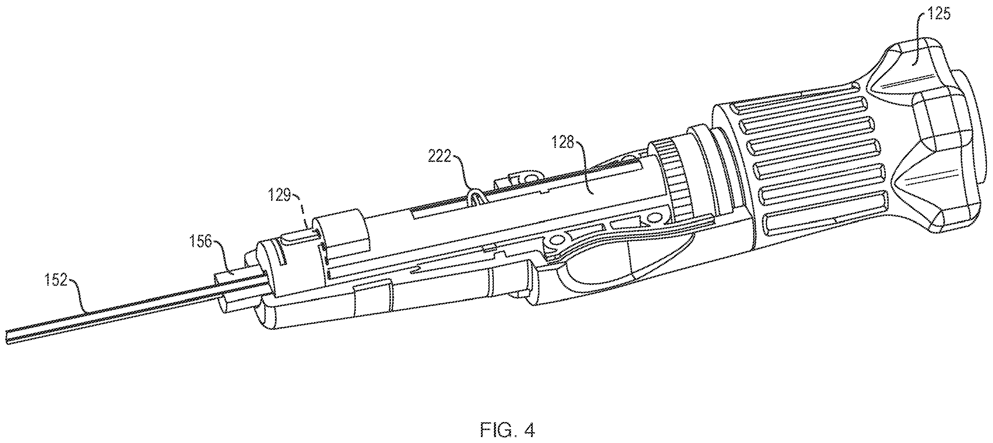

While shown as separate stages of anchor deployment in FIG. 3B and 3C, actuation means may be operatively coupled to both the slotted tube 152 and deployment member 220. With reference to FIG. 4, actuation means 125, may be coupled to a driver block 128, driver block 128 coupled to slotted tube 152. FIG. 4 shows the instrument 110 with a portion of the handle and the hub 130 removed, for simplification of understanding of the actuation mechanism. Actuation means 125 may be threadably coupled to driver block 128. Actuation of actuation means 125 such as rotation of actuation knob 126 may axially retract driver block 128 which axially retracts slotted tube 152 to the position shown in FIG. 3B. Tension on the deployment member 220 may be delayed via a slack loop 222 formed along deployment member 220 formed during assembly of the deployment member. Slack loop 222 may be sized such that initial actuation to axially retract the driver block 128 retracts the slotted tube 152 a length sufficient to expose the anchor 200 before the slack loop 222 becomes taught. A frangible portion 129 of the drivers block (shown as a dotted line) may then break, thereby halting any further retraction of the drivers block 128. Continued activation of the actuation means may solely apply tension on the deployment member 220 and expand the anchor 200 within the tissue, as shown in FIG. 3C. At a distal end of driver block 128 is a hub cover key 156 that also retracts, as will be explained hereafter.

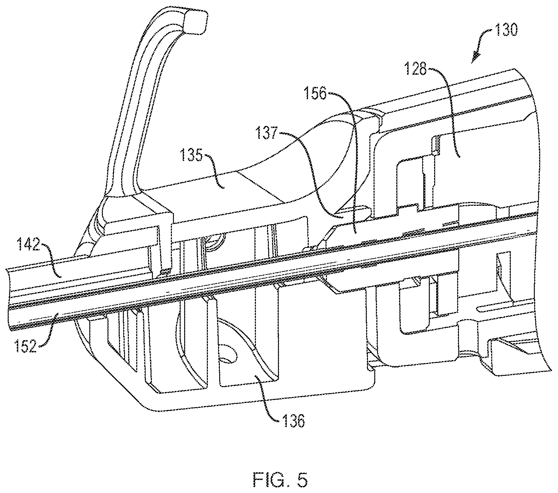

FIG. 5 illustrates a cross section of the hub 130 including the cover 1 35 and cavity 136. Cavity 136 houses the flexible repair member 250 and coupling means attached thereto. Repair member 250 and coupling means are omitted from this figure for simplification of understanding only. Slotted tube 152 extends through cavity 136. A proximal end of cover 135 may include a slot 137 to receive hub cover key 156, therein, When hub cover key 156 is disposed within slot 137, cover 135 is locked in a closed position. Hub cover key 156 is operatively coupled to the tube 152 (or drivers block 128) such that actuation means retracts the hub cover key 156 as the tube 152 retracts. This then releases the hold on the cover 135 allowing it to be removed to gain access to the repair members 250 and coupling means.

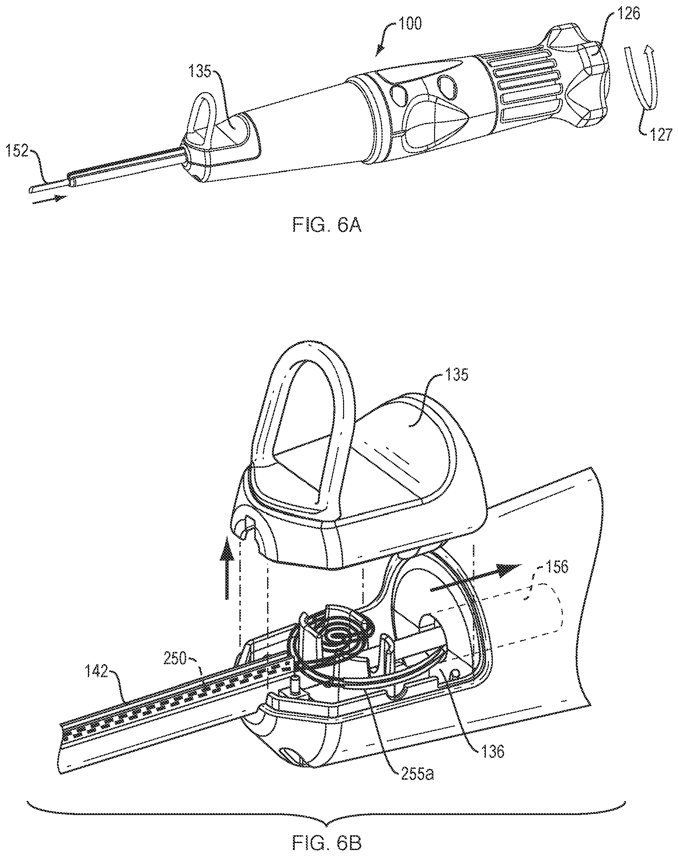

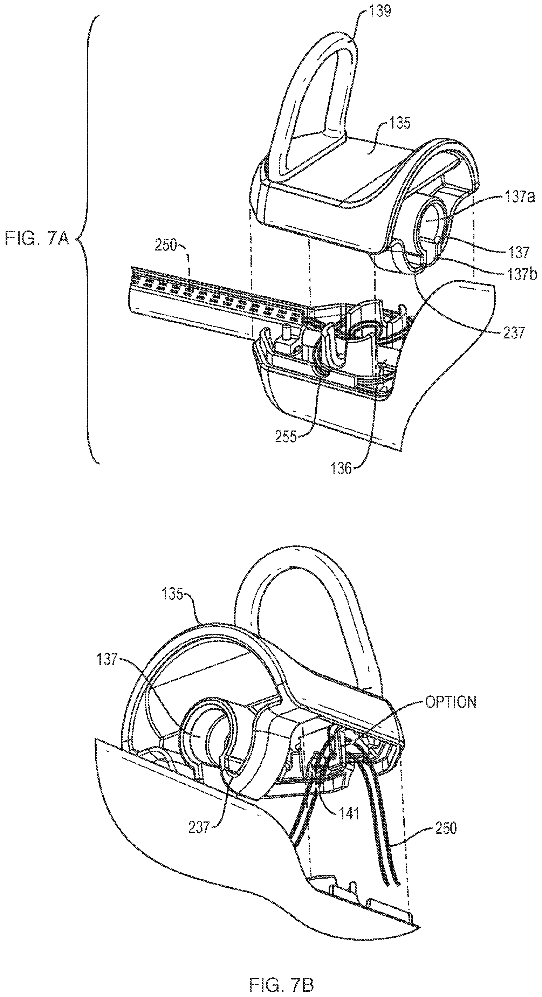

FIG. 6A and 6B illustrate an example method of anchor deployment and release of cover 135. In FIG. 6A, actuation member may be a rotation knob 126. Rotation 127 may first axially retract the tube 152 to release the anchor (as shown in FIG. 3A, 3B). Rotation 127 may also axially retracts the key 156 out of cover slot 137. Slot is seen also in FIG. 7A. Key 156 may have an axial length sufficient to remain engaged with slot 137 until most of the tube 152 is retracted. Key 156 may have an axial length such that cover is unlocked once the anchor deployment has initiated. As shown in FIG. 6B, once key 156 is axially retracted into handle housing, cover 135 may be removed to access repair member(s) 250 and coupling means 255 housed within cavity 136.

Repair members 250 extend along channel 142 from the anchor 200 and into the cavity 136. Channel 142 is an external channel that may extend from a distal most edge of shaft 140 up to cavity 136. Channel 142 may loosely hold lengths of the repair member(s) 250. Once cover 135 is removed, the user may readily access the coupling means 255 and withdraw repair member(s) 250 from both the cavity 136 and channel 142. Repair member(s) 250 may be of smaller diameter than deployment member 220. For example, repair member(s) may be #0 Ultrabraid. Deployment member 220 may be a flexible member that may be #1 Ultrabraid suture, or any suture defined between #0-#3 according to USP designations. Deploying member 220 is preferably configured to withstand the tension thereon required to deploy the anchor 200. Repair member 250 may be a #0 Ultrabraid suture and may be provided with needles attached thereto. Repair member(s) 250 may couple the anchor within both the first tissue and second tissue, such as extremity tissue. It is therefore preferable that repair member 250 may be a flexible member that is small in profile suture such as 2.0 or #0 according to USP designations. Repair member(s) 250 may define two limbs extending proximally from the anchor 200. In some embodiments, there may be more than one repair member 250. Each limb of the repair member(s) 250 may be separately pre-attached to a separate coupling means. For example if there are two repair members 250, each repair member having two limbs, then four limbs may extend along channel 142 and be housed in the cavity 136. Furthermore, each of the four limbs may have a needle attached thereto, such that four needles are also housed within cavity 136. In another example embodiments, if there are two repair members 250, each repair member having two limbs, then four limbs may extend along channel 142 and be housed in the cavity 136. In this example where the coupling means includes a cortical anchor, all four limbs may be pass through a single cortical anchor. In some example embodiments, the repair member may form a longitudinal locking passage therealong, similar to a finger trap that may knotlessly lock the coupling means to a second tissue. In other example embodiments, one flexible member such as the deployment member may be suture tape while other flexible members such as the repair member may be a suture with a braided or a hollow core, for example. Each flexible member may include markings to identify each individual flexible member during the tissue repair. For example, the repair members 250 may have a different color or series of markings therealong than the deployment member.

FIG. 7A and 7B show various views of the cover 135 and slot 137. Slot 135 may define a first segment 137a configured to wrap around the key 156 and a second segment 137b extending from the first segment 137a configured to prevent key 156 from passage therethrough. As shown first segment 137 defines a larger opening that may nest or wrap around key 156 and allow the key 156 to axially slide therethrough. Second segment 137b defines a narrow channel that extends up to an edge surface of the cover, sized to allow passage of the slotted tube 152 therethrough but not the key 156. In operation, until the key 156 is axially retracted, the cover 156 is locked onto the hub 130. Once the key 156 is axially retracted, the tube 152 is disposed within the slot 137 and the cover may be removed. A tab 139 may be pulled to remove cover 135. As shown, cover 135 is completely removable from hub 130. In other configurations, cover 135 may be hingedly attached, via for example a living hinge, and cover 135 may be rotated away from hub 130 to expose the repair members 250 and coupling means 255.

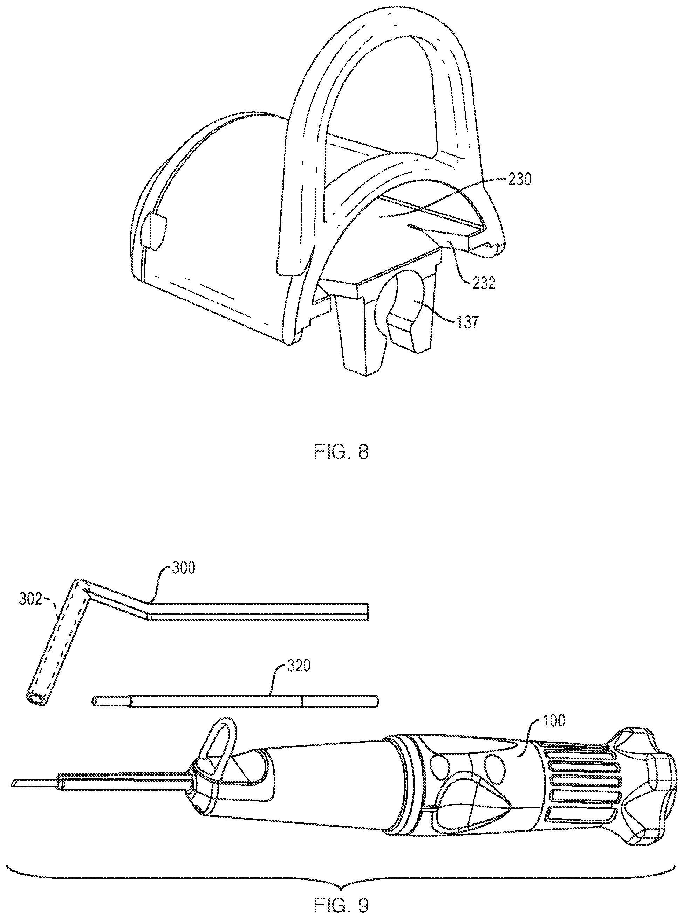

FIG. 7B illustrates cover 135 with slot 137 and may include an optional suture management slot 141. Slots 141 may loosely capture a portion of repair member(s) 250, such that upon removal of cover 135, the repair member(s) 250 are drawn out of the cavity 136. In some example embodiments, a second positive engagement means may be formed between cover 135 and hub 130 that maintains the cover 135 in position until the user grabs the tab 139. For example, slot 137 may be bounded by at least one resilient leg 237 (two shown) that is configured to flex to release cover 135. Stated in another way, second segment 137b may define a slot gap slightly smaller than a maximum diameter of tube 152, such that removal of cover requires flexing at least one leg 237 to temporarily increase this gap. Some deformation of leg(s) 237 may be required to remove cover 135. In other examples, the second positive engagement means may include a frangible portion that couples the cover 135 to the hub 130. For example, a weak ultrasonic weld or spot of adhesive may be applied to a portion between the hub 130 and cover 135 during assembly. This frangible portion must be broken to remove cover 135. As way of another example, at least one of the cover 135 and hub 130 may include at least one post that frictionally fits within an aperture or channel within the other of the at least one of the cover 135 and hub 130. The frictional fit must be overcome to remove the cover 135. Another example may include a snap fit between the cover 135 and hub 130. Cover 135 may therefore be unlocked during anchor deployment when the key 156 is axially retracted, but may need an external force from the user to overcome the second positive engagement means and remove the cover 135 from the hub 130. This may ensure that the hub does not inadvertently drop off during deployment and into the surgical field. In other example embodiments illustrated in FIG. 8, cover 135 may include a cavity 230 that houses the coupling means and a portion of the repair member(s) 250, Cavity 230 may include notches or cleats 232 that allow passage of the repair member(s) 250 therethough and may slightly pinch the repair members 250. In this example, removal of the cover 135 may concurrently remove the repair member(s) and coupling means 255 form the instrument 110.

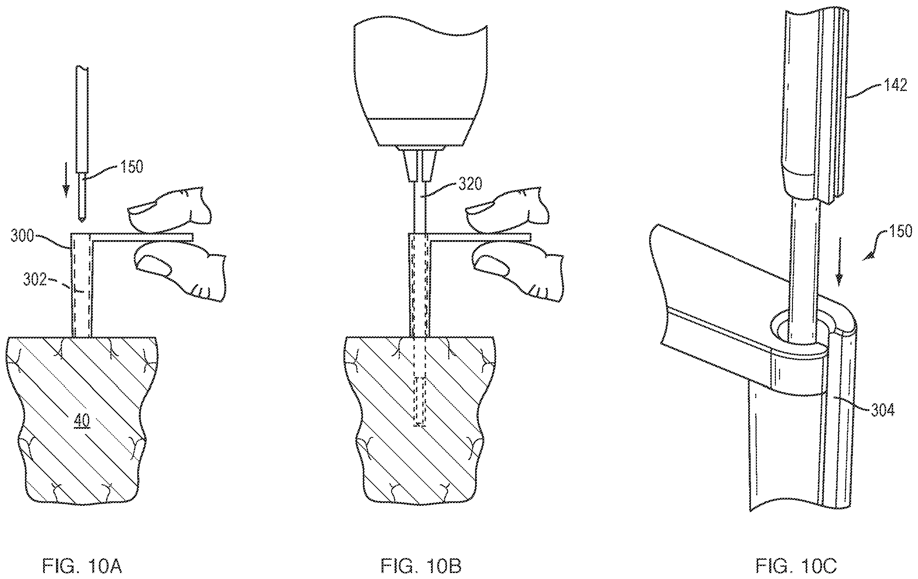

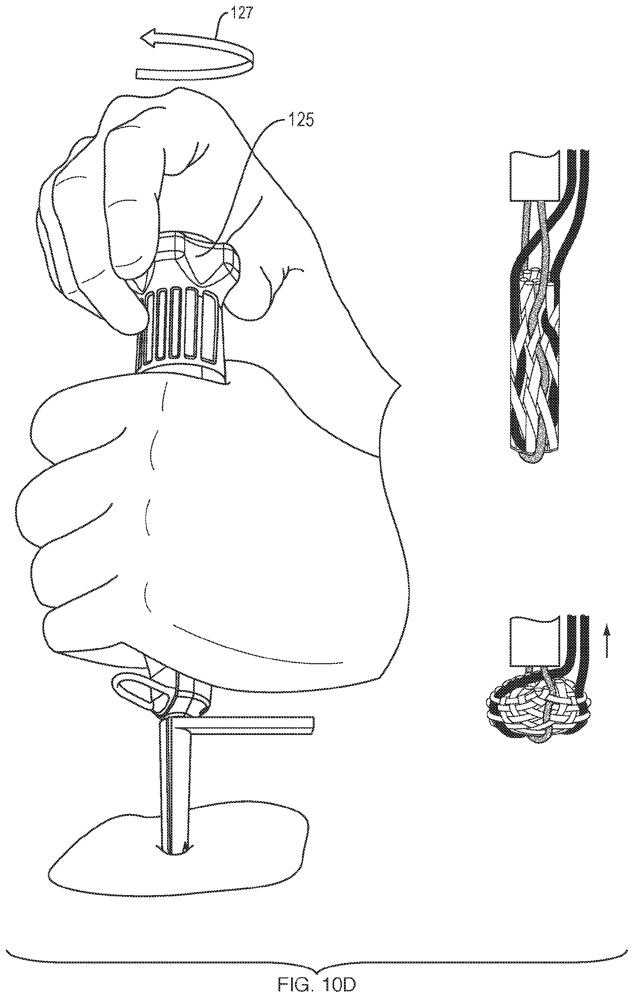

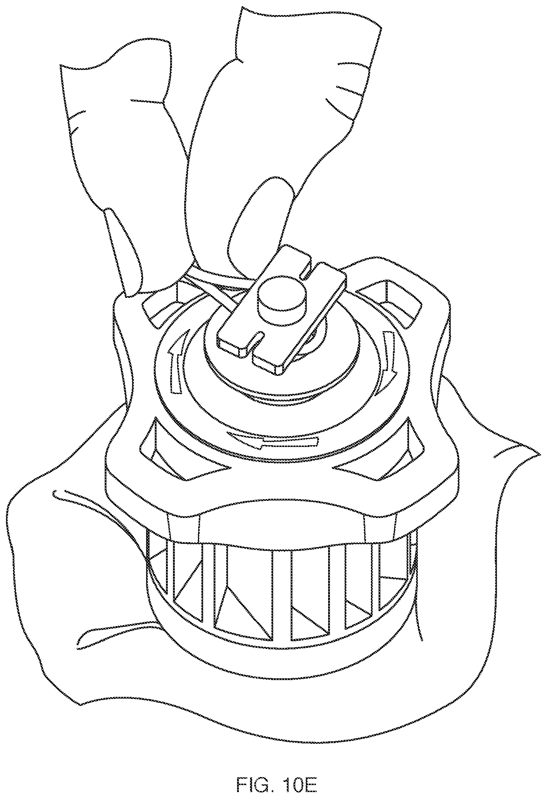

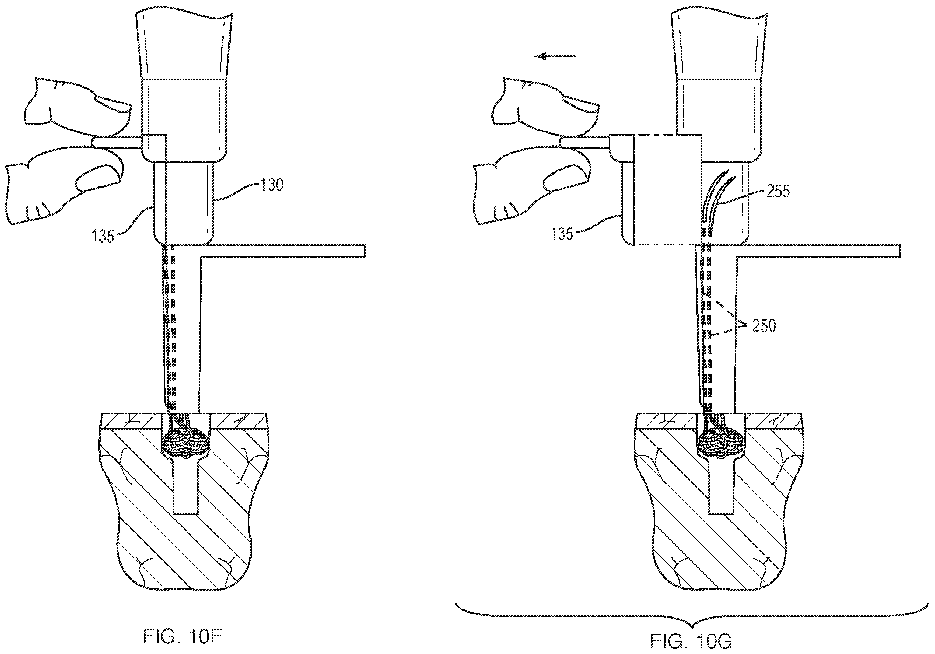

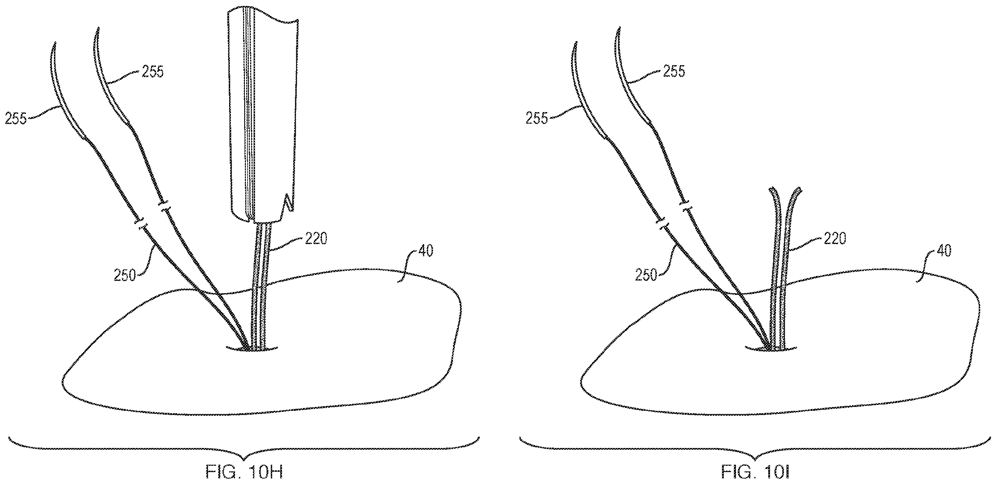

FIG. 9 shows an example kit including the tissue repair system 100, a disposable guide 300 and a drill 320. Disposable guide 300 may include a cannulation 302 for receiving distal end 150 of system 100 therethrough. FIGS. 10A-10D illustrate a method of repairing a tissue of an extremity, using the tissue repair system 100 as disclosed herein. Guide 300 may be placed at an insertion site. Guide 300 is configured to receive and guide a drill 320 to drill a tunnel in a first tissue such as bone tissue 40. Guide length may limit the depth of hole drilled into the tissue 40. Guide 300 is also configured to receive a working end 150 of system 100. Guide 300 may include an elongate slot 304 for receiving the channel 142. Guide slot 304 is configured to align channel 142 of the inserter, best illustrated in FIG. 10B. Insertion instrument 110 may include a surface that may be a distal most surface of an inserter handle hub 130 that bottoms out on disposable guide 300 when the insertion instrument is fully inserted into the tissue, to limit insertion depth, illustrated in FIG. 10C. Remaining on this figure, inserter handle actuator 125 may then be actuated to deploy the anchor 200. Deployment member may then be removed from the inserter actuator 125 to release the deployment member (FIG. 10D). In other example methods, deployment member may include a predetermined frangible portion therealong, which breaks under high tension, releasing the deployment member from the anchor. Actuation of the actuator 125 may also unlock or release a cover 135 of the hub 130. Cover 135 may then be removed to gain access to repair members 250 and coupling means 255. In an example method, coupling means may include needles 255. Removing the inserter and guide may now leave the deployed anchor in the target tissue 40 with the repair members 250 coupled to coupling means 255 extending from the anchor 200 (FIG. 10F). Deployment member 220 may be entirely removed from anchor once the anchor 200 is deployed. Alternatively, deployment member 220 may be trimmed.

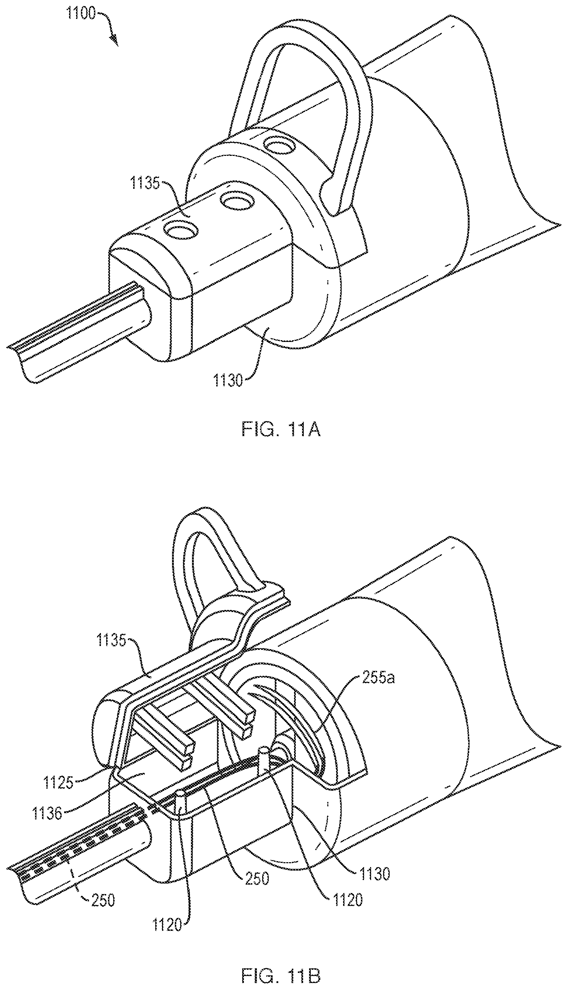

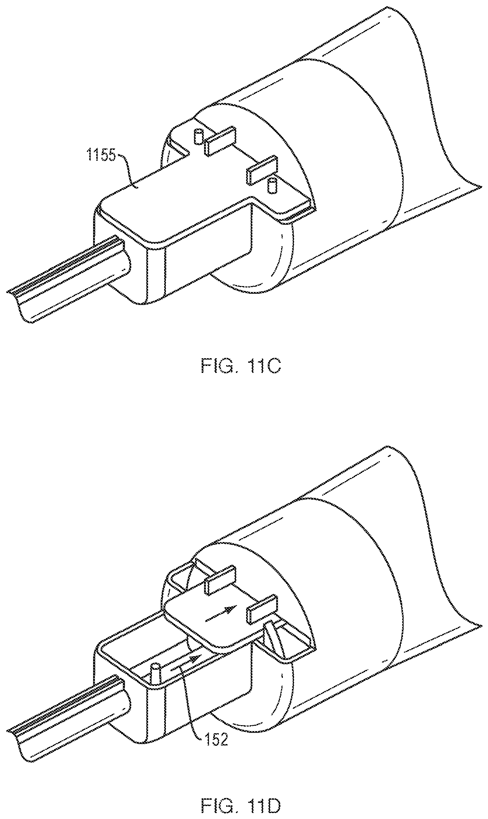

FIG. 11A shows alternative system 1100 wherein the cover 1135 may be hingedly connected to hub 1130. Hinge 1125 may extend along a lateral side of hub 1130, parallel to longitudinal axis of hub 1130. Hinge 1125 may define a living hinge. In this example embodiment, needles 255 are shown oriented traverse the hub longitudinal axis. Cavity 1136 also includes a plurality of posts 1120 that repair member(s) 250 may wrap around while stored within the cavity 1136. Posts 1120 may be disposed adjacent a periphery of the cavity 1136 so that the repair member(s) 250 forms a large loop around the posts 1120. Four posts 1120 may be disposed relative to each other, defining vertices of a quadrilateral shaped loop of repair member(s) 250. As shown, two post 1120 may be disposed relative to each other, defining two opposing ends of a wrap loop.

FIG. 11B illustrates another embodiment in a closed and open configuration where the cover 1155 is axially slideable. Cover 1155 may be directly coupled to the tube 152, such that axially retracting the tube 152 axially retracts the cover 1155. The cover 1155 may slide into a slot in the handle to expose the repair member(s) 250 and coupling means 255 (not shown in this figure)

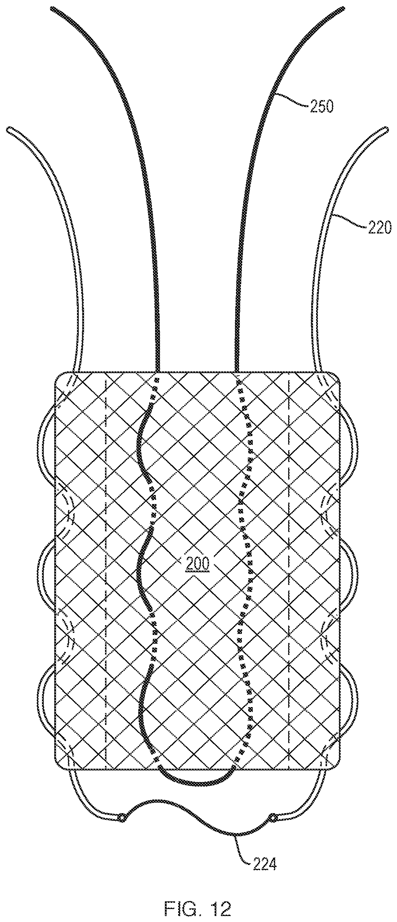

FIG. 12 illustrates an example soft anchor 200 with a deployment member 220 interwoven therethrough and a repair member 250 coupled thereto. Example soft anchor 200 may be tubular. The repair member 250 as shown may be coupled in a variety of ways, as disclosed herein. In this example, the repair member 250 is coupled along a path, parallel to the deployment member 220, but circumferentially offset from the path of the deployment member 220. Deployment member 220 may include a frangible portion 224. As shown, this portion is at a distal end of anchor 200. However, this frangible portion 224 may be disposed anywhere along the deployment member 220. Frangible portion 224 may be formed by a variety of means. Frangible portion 224 may be formed by weakening a portion of the deployment member 220. Weakening may be via cutting, fraying or damaging the deployment member. In other embodiments, the deployment member 224 may be formed as two separate lengths of a flexible member coupled with a weak link at the frangible portion 224. For example, a two ends of the two separate flexible member lengths may extend through a tubular clip or crimp. Frangible portion 224 may be formed during the braiding process and braids may be omitted during a short portion of the length of the flexible member.

Frangible portion 224 is configured to break at a predetermined tension. This predetermined tension is at or above the tension required to deploy the anchor 200. Upon breaking, the deployment member 220 may be drawn out of the anchor 200 and removed from the tissue. Once the anchor 200 is deployed, continued tension on the anchor is no longer necessary to maintain an expanded configuration of the anchor 200. The deployment member 220 therefore may not be part of the final repair construct. This reduces the profile of the final repair construct at the end of the procedure. Due to the smaller and finer tissues of the extremities, a minimal profile repair construct is preferable. Given that the deployment member 220 may be a large diameter suture, relative to the repair member(s) 250, removal of this larger diameter element reduces the bulkiness of the final repair construct significantly.

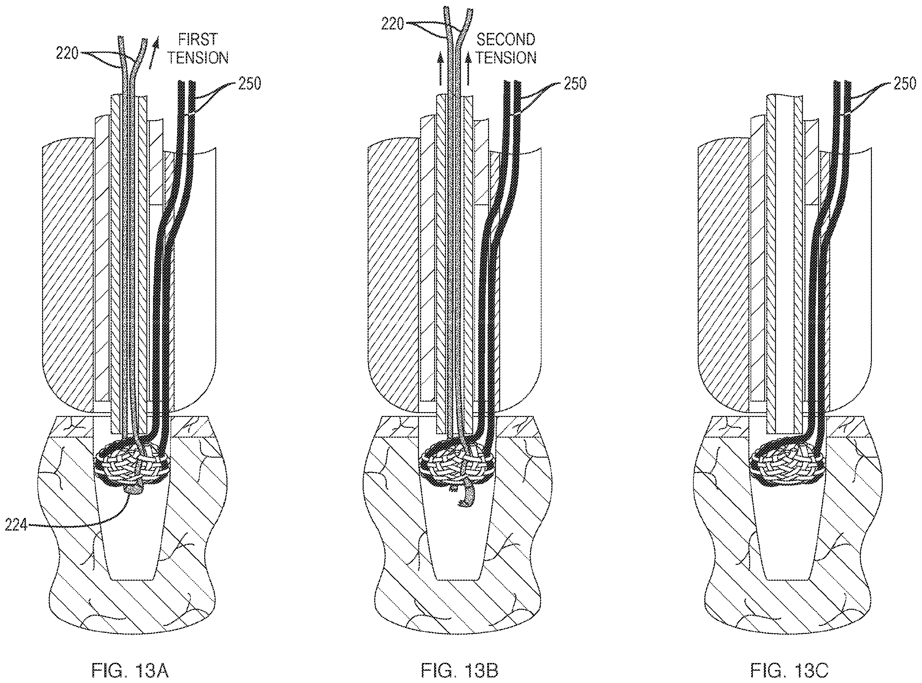

A method of coupling a first tissue to a second tissue may therefore include inserting an anchor into a first tissue, and deploying the anchor to an expanded configuration within the tissue, with a deployment member (FIG. 13A). Deploying the anchor includes tensioning a deploying member operatively coupled to the anchor. Tensioning may include tensioning a deploying member to a first predetermined tension to deploy the anchor. The method may then include applying tension to the deploying member at a second predetermined tension that is at or above the first predetermined tension. The second predetermined tension is configured to break the deploying member at a predetermined frangible portion 224 along the deploying member (FIG. 13B). The deploying member may then be detached from the anchor and thereby removed from the anchor (FIG. 13C). The first predetermined tension may be 120N. The second predetermined tension may be 140N. A repair suture and connecting means operatively coupled to the anchor may then couple the anchor to a second tissue. The repair suture may be stored and covered in an anchor insertion instrument, the cover being locked in a covered position as provided. While deploying the anchor, the cover lock is released concomitantly with deployment.

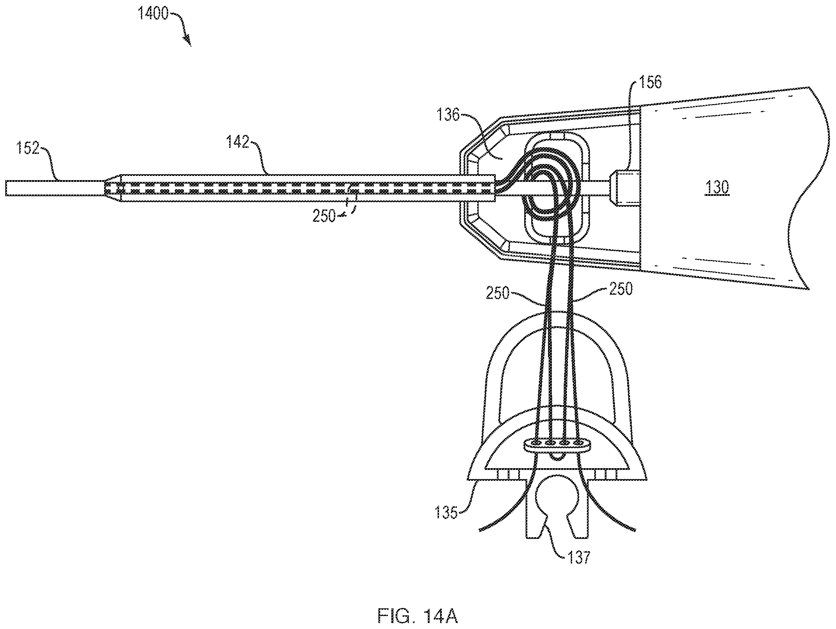

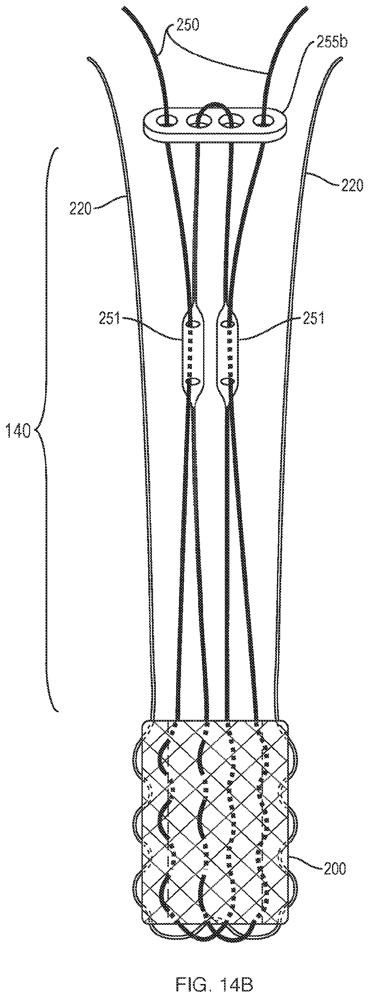

In some example embodiments, the coupling means 255 may be at least one needle 255a. In other example embodiments, the coupling means may include a second anchor 255b. The second anchor 255b may be different from the first anchor 200. The second anchor 255b may be a cortical button style anchor. Shown in FIG. 14A is a tissue repair system 1400 similar to system 100 with an anchor housed within a slotted tube 152 (obscured by the tube 152). Like components have been given the same reference numerals, where the elements or components are the same as in system 100. The repair member 250 (or members) is operatively coupled to both anchor 200 and anchor 255b at opposing ends and also extends along the channel 142. Second anchor 255b may be stored within either the cover 135 or cavity 136. As shown, anchor 255b may be stored within cover 136 and removal of the cover simultaneously removes the anchor 255b and a portion of the repair member 250. FIG. 14A illustrates the cover 136 separated from the hub 130, for explanation purposes of the figure. However, similar to previous embodiments, given the position of the tube 152 and key 156, in reality the cover 136 would be locked onto the hub 130. In some example embodiments, repair member (or members) 250 may form a plurality of loops that extend between the anchor 200 and anchor 255b, and therefore may extend along channel 142 multiple times. This is represented in FIG. 14B. In some example embodiments, the repair member 250 extends through itself for a length, forming a locking passage 251 (finger trap or splice). Locking passage 251 may also be stored within cavity 136. Shown in FIG. 14B, the repair member 250 may extend along the entire length of anchor 200. In other example embodiments, the repair member 250 may only extend through a portion of the anchor 200. This may make reduction of the any loops formed by the repair member 250 easier and also reduce bulk through the anchor 200.

A method of securing a first tissue to a second tissue may include inserting a tissue anchor into the first tissue, the inserting with an instrument including a handle at a proximal end, a shaft extending distally from the handle and a hub with a cover therebetween. An actuation member of the instrument may be moved or actuated to deploy the tissue anchor and fix the tissue anchor with the first tissue. Actuation may include rotating a portion of the handle. Deploying the tissue anchor may include expanding the anchor within the tissue. Deploying the anchor may include deforming the anchor. Deploying may include re-orienting the anchor. Deploying the anchor may include applying tension to a deployment member operatively coupled to the anchor. Deploying the anchor may include axially moving a portion of the instrument. Deploying the anchor may include axially retracting a tube of the instrument, a distal end of the tube configured to house the anchor during insertion. Deploying may concomitantly unlock the housing cover. Deploying may axially retract a tube to concomitantly expose the tissue anchor to the first tissue and unlock the housing cover.

The housing cover may then be removed to expose a coupling means and a repair member disposed within the housing. The repair member may extend along an external portion of the shaft along a channel. The repair member is operatively coupled to the tissue anchor. The repair member may interweave through the tissue anchor. Unlocking the cover may release the cover to fall away from the hub. Unlocking the cover may unlock the cover only, and removing the cover to expose the repair member and coupling means may further require overcoming a secondary engagement means between the hub and cover. Removing may include breaking a weld or adhesive, removing may include hingedly opening the cover. Removing may include overcoming a friction or snap fit. Removing may expose the repair member and coupling means housed within the hub. Removing the cover may draw the repair member and/or coupling means out of the hub.

The coupling means may be a needle 255a, and the repair member may define two limbs, each limb having a needle attached thereto and houses within the cover. The repair member may then be coupled to a second tissue by piercing the second tissue using the needle or needles. In another example method, the coupling means may be a repair anchor 255b that may be different from the tissue anchor. The repair anchor may be a cortical button. The repair member may form a plurality of loops between the tissue anchor and the repair anchor, with two limbs extending from the repair anchor 255b. The repair anchor and repair member may be inserted through a hole in the second tissue or around the second tissue to couple the repair anchor to the second tissue and thereby the first tissue to the second tissue. Drawing on the two limbs may reduce a size of the plurality of loops and draw the second and first tissue towards each other. At least one locking passage may be formed along a length of the repair member, such that tension on the repair member may knotlessly lock the locking passage and lock the first and second tissue in place. The locking passage may also be provided stored within the hub.

The deploying member may include a frangible portion, and deploying may include tensioning the deploying member to deploying the anchor followed by tensioning the deploying member to break the frangible portion and then removing the deploying member. The deploying member may interweave through the tissue anchor, and the frangible portion may be adjacent the tissue anchor. In other embodiments, the instrument may include a cutting edge that may be separately activated. After the anchor is deployed, the cutting edge may be engaged to transect a portion of the deploying member. The tissue anchor may be formed of a soft material and may be formed of braided suture. Removing the deploying member leaves the tissue anchor fixed with the first tissue in the deployed configuration.

One skilled in the art will realize the disclosure may be embodied in other specific forms without departing from the essential characteristics thereof. The foregoing examples are therefore to be considered in all respects illustrative rather than limiting of the disclosure described herein. Scope of the disclosure is thus indicated by the appended claims, rather than by the foregoing description.

## Claims

1. A tissue repair system (100) for securing a first tissue to a second tissue, comprising:
an instrument (110) including a handle (120) at a proximal end, a shaft (140) extending distally from the handle, the instrument (110) also including a housing (130) with a cover (135);
a tissue anchor (200) disposed at a distal end (150) of the shaft (140), the tissue anchor (200) operatively coupled to an actuation member (125) of the handle (120) via a deployment member (220); and
a repair member (250), configured to couple the tissue anchor (200) to the second tissue, the repair member (250) defining a first end operatively coupled to the tissue anchor (200) and second end operatively coupled to at least one coupling means (255); the second end and coupling means (255) housed within the instrument housing (130) and wherein actuation of the actuation member (125) is configured to release the housing cover (135) for access to the coupling means;
**characterized in that** the actuation of the actuation member (125) is also configured to deploy the tissue anchor (200) via the deployment member (220) and thereby couple the tissue anchor (200) with the first tissue.

2. The tissue repair system of claim 1 wherein the shaft has a first position and second position, wherein in the first position the shaft is operatively coupled to the housing cover to lock the housing cover and wherein actuation of the actuation member moves the shaft to the second position that releases the housing cover.

3. The tissue repair system of claim 2 wherein the shaft distal end is configured to house the tissue anchor therein, and wherein actuation of the actuation member axially moves the shaft to expose the tissue anchor.

4. The tissue repair system of claim 1 wherein the housing cover defines an external circumferential surface the instrument.

5. The tissue repair system of claim 1 wherein the housing cover includes a slot for engaging the repair member, such that removing the housing cover removes a portion of the repair member.

6. The tissue repair system of claim 1 wherein the coupling means is selected from a group comprising a needle, a threading member, a cortical button and another tissue anchor.

7. The tissue repair system of claim 1, wherein the tissue anchor is a soft anchor and the deployment member includes a flexible member repeatedly interwoven through the soft anchor.

8. The tissue repair system of claim 7 wherein the flexible member defines a first cross section and the repair member defines another flexible member having a second cross section, smaller than the first cross section.

9. The tissue repair system of claim 7 wherein the flexible member includes a frangible portion configured to fracture at a predetermined value indicative of tissue anchor deployment.

10. The tissue repair system of claim 1 wherein the repair member includes at least two flexible members, defining at least four flexible member limbs, each limb terminating with a coupling means that is a needle.

11. The tissue repair system of claim 1,
wherein the repair member (250) is a flexible member, and wherein actuation of the actuation member is configured to axially move the shaft.

## Patentansprüche

1. Gewebereparatursystem (100) zum Befestigen eines ersten Gewebes an einem zweiten Gewebe, umfassend:
ein Instrument (110), das einen Griff (120) an einem proximalen Ende und einen sich distal von dem Griff erstreckenden Schaft (140) aufweist, wobei das Instrument (110) auch ein Gehäuse (130) mit einer Abdeckung (135) aufweist,
einen Gewebeanker (200), der an einem distalen Ende (150) des Schafts (140) angeordnet ist, wobei der Gewebeanker (200) über ein Ablegeglied (220) an ein Betätigungsglied (125) des Griffs (120) wirkgekoppelt ist, und
ein Reparaturglied (250), das dazu ausgestaltet ist, den Gewebeanker (200) an das zweite Gewebe zu koppeln, wobei das Reparaturglied (250) ein an den Gewebeanker (200) wirkgekoppeltes erstes Ende und ein an mindestens ein Kopplungsmittel (255) wirkgekoppeltes zweites Ende definiert, wobei das zweite Ende und Kopplungsmittel (255) in dem Instrumentengehäuse (130) untergebracht sind und wobei die Betätigung des Betätigungsglieds (125) dazu ausgestaltet ist, die Gehäuseabdeckung (135) für den Zugriff auf das Kopplungsmittel freizugeben,
**dadurch gekennzeichnet, dass** die Betätigung des Betätigungsglieds (125) auch dazu ausgestaltet ist, den Gewebeanker (200) über das Ablegeglied (220) abzulegen und dadurch den Gewebeanker (200) an das erste Gewebe zu koppeln.

2. Gewebereparatursystem nach Anspruch 1, wobei der Schaft eine erste Position und eine zweite Position aufweist, wobei der Schaft in der ersten Position an die Gehäuseabdeckung wirkgekoppelt ist, um die Gehäuseabdeckung zu verriegeln, und wobei der Schaft durch eine Betätigung des Betätigungsglieds in die zweite Position bewegt wird, die die Gehäuseabdeckung freigibt.

3. Gewebereparatursystem nach Anspruch 2, wobei das distale Ende des Schafts zur Aufnahme des Gewebeankers darin ausgestaltet ist und wobei der Schaft durch eine Betätigung des Betätigungsglieds axial bewegt wird, um den Gewebeanker freizulegen.

4. Gewebereparatursystem nach Anspruch 1, wobei die Gehäuseabdeckung eine äußere Umfangsfläche des Instruments definiert.

5. Gewebereparatursystem nach Anspruch 1, wobei die Gehäuseabdeckung einen Schlitz zur Ineingriffnahme des Reparaturglieds aufweist, so dass durch das Entfernen der Gehäuseabdeckung ein Abschnitt des Reparaturglieds entfernt wird.

6. Gewebereparatursystem nach Anspruch 1, wobei das Kopplungsmittel aus einer Gruppe ausgewählt ist, die eine Nadel, ein Einfädelglied, einen Kortikalbutton und einen weiteren Gewebeanker umfasst.

7. Gewebereparatursystem nach Anspruch 1, wobei der Gewebeanker ein weicher Anker ist und das Ablegeglied ein flexibles Glied aufweist, das wiederholt durch den weichen Anker verwoben ist.

8. Gewebereparatursystem nach Anspruch 7, wobei das flexible Glied einen ersten Querschnitt definiert und das Reparaturglied ein weiteres flexibles Glied definiert, das einen zweiten Querschnitt aufweist, der kleiner als der erste Querschnitt ist.

9. Gewebereparatursystem nach Anspruch 7, wobei das flexible Glied einen zerbrechbaren Abschnitt aufweist, der zum Brechen bei einem vorbestimmten Wert ausgestaltet ist, der ein Ablegen des Gewebeankers anzeigt.

10. Gewebereparatursystem nach Anspruch 1, wobei das Reparaturglied mindestens zwei flexible Glieder umfasst, die mindestens vier Schenkel des flexiblen Glieds definieren, wobei jeder Schenkel mit einem Kopplungsmittel endet, das eine Nadel ist.

11. Gewebereparatursystem nach Anspruch 1,
wobei das Reparaturglied (250) ein flexibles Glied ist und wobei eine Betätigung des Betätigungsglieds zum axialen Bewegen des Schafts ausgestaltet ist.

## Revendications

1. Système de réparation tissulaire (100) pour fixer un premier tissu à un second tissu, comprenant :
un instrument (110) comportant un manche (120) à une extrémité proximale, un arbre (140) s'étendant distalement à partir du manche, l'instrument (110) comportant également un boîtier (130) avec un couvercle (135) ;
un ancrage tissulaire (200) disposé à une extrémité distale (150) de la tige (140), l'ancrage tissulaire (200) étant accouplé fonctionnellement à un élément d'actionnement (125) du manche (120) par l'intermédiaire d'un élément de déploiement (220) ; et
un élément de réparation (250), configuré pour accoupler l'ancrage tissulaire (200) au second tissu, l'élément de réparation (250) définissant une première extrémité accouplée fonctionnellement à l'ancrage tissulaire (200) et une seconde extrémité accouplée fonctionnellement à au moins un moyen d'accouplement (255) ; la seconde extrémité et un moyen d'accouplement (255) étant logés dans le boîtier d'instrument (130) et dans lequel l'actionnement de l'élément d'actionnement (125) est configuré pour libérer le couvercle du boîtier (135) pour accéder au moyen d'accouplement ;
**caractérisé en ce que** l'actionnement de l'organe d'actionnement (125) est également configuré pour déployer l'ancrage tissulaire (200) par l'intermédiaire de l'élément de déploiement (220) et ainsi accoupler l'ancrage tissulaire (200) au premier tissu.

2. Système de réparation tissulaire selon la revendication 1 dans lequel l'arbre a une première position et une seconde position, dans lequel, dans la première position, l'arbre est accouplé fonctionnellement au couvercle de logement pour verrouiller le couvercle de logement et dans lequel l'actionnement de l'élément d'actionnement déplace l'arbre vers la seconde position qui libère le couvercle de logement.

3. Système de réparation tissulaire selon la revendication 2 dans lequel l'extrémité distale de l'arbre est configurée pour loger l'ancrage tissulaire dans celle-ci, et dans lequel l'actionnement de l'élément d'actionnement déplace axialement l'arbre pour faire apparaître l'ancrage tissulaire.

4. Système de réparation tissulaire selon la revendication 1 dans lequel le couvercle de logement définit une surface circonférentielle externe de l'instrument.

5. Système de réparation tissulaire selon la revendication 1 dans lequel le couvercle de logement comporte une fente pour une mise en prise avec l'élément de réparation, de telle sorte que le retrait du couvercle de logement enlève une partie de l'élément de réparation.

6. Système de réparation tissulaire selon la revendication 1 dans lequel le moyen d'accouplement est choisi dans un groupe comprenant une aiguille, un élément d'enfilage, un bouton cortical et un autre ancrage tissulaire.

7. Système de réparation tissulaire selon la revendication 1, dans lequel l'ancrage tissulaire est un ancrage souple et l'élément de déploiement comporte un élément flexible entrelacé de manière répétée à travers l'ancrage souple.

8. Système de réparation tissulaire selon la revendication 7 dans lequel l'élément flexible définit une première section transversale et l'élément de réparation définit un autre élément flexible ayant une seconde section transversale, plus petite que la première section transversale.

9. Système de réparation tissulaire selon la revendication 7 dans lequel l'élément flexible comprend une partie cassable configurée pour se fracturer à une valeur prédéterminée indiquant un déploiement d'ancrage tissulaire.

10. Système de réparation tissulaire selon la revendication 1 dans lequel l'élément de réparation comprend au moins deux éléments flexibles, définissant au moins quatre membres flexibles, chaque membre se terminant par un moyen d'accouplement qui est une aiguille.

11. Système de réparation tissulaire selon la revendication 1,
dans lequel l'élément de réparation (250) est un élément flexible, et dans lequel l'actionnement de l'élément d'actionnement est configuré pour déplacer axialement l'arbre.
